# EUROPEAN PATENT SPECIFICATION

(11) **EP 4 319 649 B1**
(45) Date of publication and mention of the grant of the patent: **02.12.2026**
(21) Application number: 22714709.7
(22) Date of filing: 16.03.2022
(51) Int. Cl.: A61B 17/06, A61B 17/04

(54) **SUTURE CONSTRUCT**
NAHTKONSTRUKT
CONSTRUCTION DE SUTURE

(30) Priority: 05.04.2021 US 202163170760 P
(43) Date of publication of application: 14.02.2024
(73) Proprietor: Biomet Manufacturing, LLC, Warsaw, IN 46582 (US)
(72) Inventor: NGUYEN, Hoang, Litchfield Park, Arizona 85340 (US); WINSLOW, Nathan A., Scottsdale, Arizona 85266 (US)
(74) Representative: Taor, Simon Edward William
(86) International application number: PCT/US2022/020612
(87) International publication number: WO 2022/216424

(56) References cited:
- CN-B- 107 374 684
- US-A1- 2007 027 475
- US-A1- 2010 217 315
- US-A1- 2018 353 167
- US-A1- 2019 099 258
- US-A1- 2020 360 009

## Description

### CLAIM OF PRIORITY

This application claims the benefit of U.S. Provisional Patent Application Serial No. 63/170,760, filed on April 5, 2021, the benefit of priority of which is claimed hereby.

### FIELD OF THE DISCLOSURE

The present subject matter relates to surgical procedures and devices and, more particularly, to prostheses, methods and systems related to soft tissue repair.

### BACKGROUND

The successful reattachment of soft tissue to other tissue and/or bone can be a significant concern, especially in the sports medicine industry. Soft tissues are often fixed to various positions on the bone. For example, to replace a natural tendon fixation point or to replace the tendon itself, fixing a graft to a selected bone area may be desired. Thus, one means to fix a soft tissue to the selected area is to provide a suture through a selected portion of the soft tissue and fix the other end of the suture to a selected area on the bone with anchor(s) and/or tack(s).

CN107374684B discloses a suture end cyclization method. A multi-stand suture is adopted, and the method includes the steps that firstly, a point close to a free end of the suture is selected and marked as a first stretching-out point, and the free end stretches out from the first stretching-out point to form a suture ring; then, a point, relatively close to the free end, on the suture ring is selected and marked as a second stretching-out point, the first stretching-out point and the second stretching-out point are located on the portions, at the two ends of the suture ring, of the suture respectively, and the free end is stretched out from the second stretching-out point; then, the suture is tightened up, so that the first stretching-out point coincides with the second stretching-out point; finally, two free ends of the suture form a new free end by threading.

US2019/099258A1 discloses an adjustable fixation device including a cleat portion and a suture portion, where the suture portion has a loop portion and a suture tail. Pulling on the suture tail places in tension a graft coupled to the loop portion. The suture is arranged to convey a mechanical advantage so that substantial force can be readily and controllably applied to the graft.

US2018/353167A1 discloses an anchor construct having an anchor having a length of suture material passing therethrough, the suture material having a loop strand terminating at a first end and a post strand terminating at a second end, a splice loop formed by first end, a sliding construct formed by the first end and the second end, wherein the sliding construct is configured to adjust the relative position of the splice loop and the anchor. The anchor construct can be deployed by passing the first end through a first body and through the splice, creating a locking loop of a first size around the first body, implanting the anchor into a bone hole, pulling the post strand to decrease the perimeter of the positioning loop to a second size smaller than the first size, and pulling the loop strand to decrease the perimeter of the locking loop to a smaller second size.

### SUMMARY

According to an aspect of the invention, there is provided a suture as set out in claim 1. Optional features are set out in claims 2 to 5.

According to another aspect of the invention, there is provided a system as set out in claim 6.

According to another aspect of the invention, there is provided a method of manufacturing a suture as set out in claim 7. Optional features are set out in claims 8 to 9.

### BRIEF DESCRIPTION OF THE FIGURES

The above-mentioned and other features and advantages of this disclosure, and the manner of attaining them, will become more apparent and the disclosure itself will be better understood by reference to the following description of embodiments taken in conjunction with the accompanying drawings, wherein:
FIG. 1 illustrates a suture in accordance with at least one example of the present disclosure.
FIG. 1A is a cross-section view of the suture of FIG. 1.
FIG. 2 is an enlarged view of a part of the suture of FIG. 1 being pierced by a needle according one example of the present disclosure.
FIG. 2A is an enlarged view of the part of the suture pierced by the needle of FIG. 2.
FIG. 3 illustrates part of the suture of FIG. 2 with a second part coupled to the needle according one example of the present disclosure.
FIG. 4 illustrates the part of the suture passed through an aperture created by the needle so as to form a loop according to one example of the present disclosure.
FIG. 5 illustrates the loop of FIG. 4 reduced to create a feature on a surface of the suture adjacent the aperture according to one example of the present disclosure.
FIG. 6 illustrates a second aperture created by the needle adjacent the aperture of FIGS. 2-5 and the suture about to be passed through the second aperture as discussed in reference to FIGS. 3-5 according to one example of the present disclosure.
FIG. 7 illustrates a plurality of apertures including the first and second apertures and a plurality of features including the feature according to one example of the present disclosure.
FIG. 8 illustrates a bead positioned in a loop created in the suture in a manner of FIG. 4 according to an example of the present application.
FIG. 8A illustrates the bead captured by a feature created when the loop is reduced according to an example of the present application.
FIG. 9 illustrates a surgical device positioned in a loop created in the suture in a manner of FIG. 4 according to an example of the present application.
FIG. 9A illustrates the surgical device captured by a feature created when the loop is reduced according to an example of the present application.
FIG. 10 illustrates a second surgical device positioned in a loop created in the suture in a manner of FIG. 4 according to an example of the present application.
FIG. 10A illustrates the second surgical device captured by a feature created when the loop is reduced according to an example of the present application.
FIG. 11 illustrates a knot positioned in a loop created in the suture in a manner of FIG. 4 according to an example of the present application.
FIG. 11A illustrates the knot captured by a feature created when the loop is reduced according to an example of the present application.
FIG. 12 shows a flowchart for a manufacturing method in accordance with at least one example of the present disclosure.

Corresponding reference characters indicate corresponding parts throughout the several views. The exemplifications set out herein illustrate exemplary embodiments of the disclosure, and such exemplifications are not to be construed as limiting the scope of the disclosure in any manner.

### DETAILED DESCRIPTION

Disclosed herein are methods, systems and devices that can facilitate a higher friction area on a suture and can facilitate a rapid connection of an element (a knot, another suture, a bead, and/or another surgical device) with the suture. The systems and devices facilitate the passage of part of the suture through an aperture created in the same suture. As a result of the suture being passed through itself, an initial loop is formed. This initial loop is used for connection to the element. The initial loop can be reduced by drawing the suture through the aperture until the loop is closed (i.e., substantially eliminated) leaving a feature comprising a bump along a surface of the suture. This surface feature can cause a higher friction area along the suture surface, and therefore, can be more inclined to contact, catch, grasp and/or hold soft tissue, for example. The surface feature can be helpful in creating a higher friction area so that the suture is less likely to slip against itself. Thus, the surface feature can decrease he likelihood of a slip of the suture, such as a suture knot slipping along a length of the suture. The surface feature can be helpful in coupling with tissue to facilitate coupling between the tissue and the suture. The size of the surface feature can be altered as desired by adding the element (the knot or the bead) to create a larger region along the suture of increased size for the feature. Multiple apertures and multiple passes of the suture through the aperture are contemplated to create regions of higher friction having multiple surface features along the longitudinal length of the suture.

The present inventors have recognized, among other things, that existing sutures can require a multiple step process for connection of the suture to tissue or for connection with another surgical device such as a suture anchor. This process can be challenging and time consuming. For example, currently known knotless suture anchor constructs typically rely on multiple components that must interact with one another precisely and in a reliable manner to facilitate anchoring of suture. Furthermore, sutures can have coatings or can be formed of material(s) that do not facilitate adequate friction with tissue to cause desired coupling (e.g., catching/grasping) of the suture with tissue or with itself (hence, the suture can possibly slip as discussed previously).

Considering these and other factors, the present inventors propose sutures and related systems and methods that can provide for faster, easier, and more reproducible surgical techniques. The suture and related systems and methods can be constructed/performed with little additional components or complex techniques but by simply using an aperture through the suture and passing a part of that suture through the aperture and reducing the initial loop to create the feature. The suture, systems and methods disclosed herein can allow a surgeon to fasten suture to tissue or couple suture to a surgical device such as an anchor without tying a knot. Again, this can simplify surgical complexity.

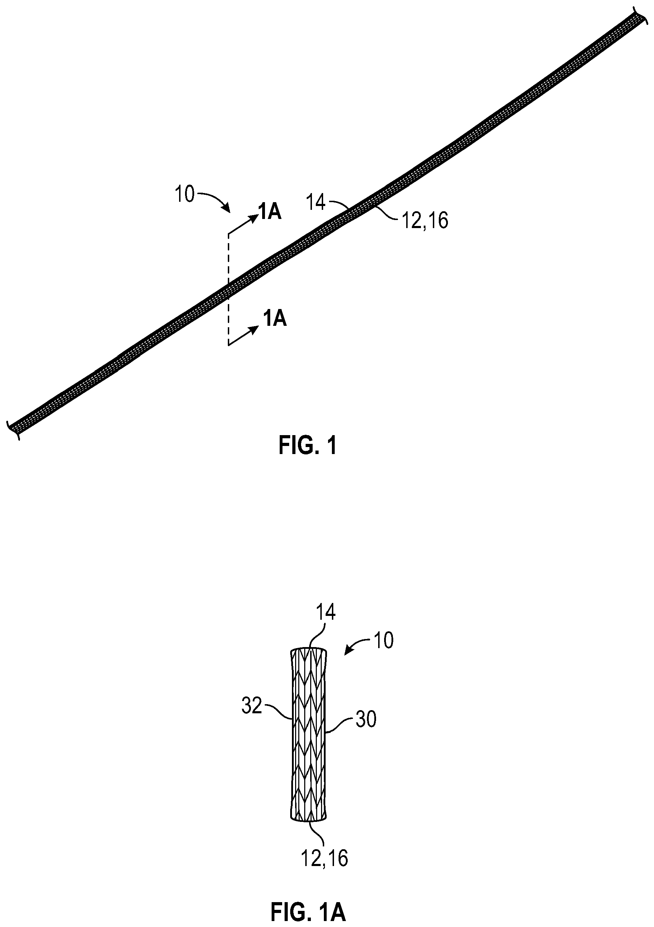

Turning now to the figures, FIGS. 1 and 1A shows a surgical suture 10 configured for connection to tissue. The suture 10 includes a body 12 having an exterior surface 14. The body 12 comprises an elongate strand 16 having a longitudinal extent along a longitudinal axis LA. The elongate strand 16 can comprise a single strand or a plurality of strands. The plurality of strands can be woven, braided or otherwise interconnected.

The suture 10 can be any type of suture known in the art. Thus, the suture 10 can comprise a multi-strand or mono-strand suture. The suture 10 can be braided or non-braided. The suture 10 can have any shape such as round, flat, broad band, tape, ribbon, etc. The suture 10 can be constructed of any material as known in the art. Examples of suitable sutures include, but are not limited to, ZipLoop^{™}, BroadBand^{™}, MaxBraid, TRU-Link and ExpressBraid^{™} sutures of Zimmer Biomet, Inc. The suture 10 can be formed of various materials such as but not limited to ultra-high-molecular weight polyethylene, other types of polyethylene, Nylon, etc., for example.

FIGS. 1 and 1A show the suture 10 is a ribbon or band type suture formed of a plurality of braids and can be flat and broad rather than round. However, round suture is also contemplated as benefiting from the constructs and techniques discussed herein. Due to the ribbon or band type configuration shown, the suture 10 can have a first side 30 that is substantially flat and an opposing second side 32 that is substantially flat as shown in FIG. 1A. The first side 30 and the second side 32 can extend generally parallel with one another.

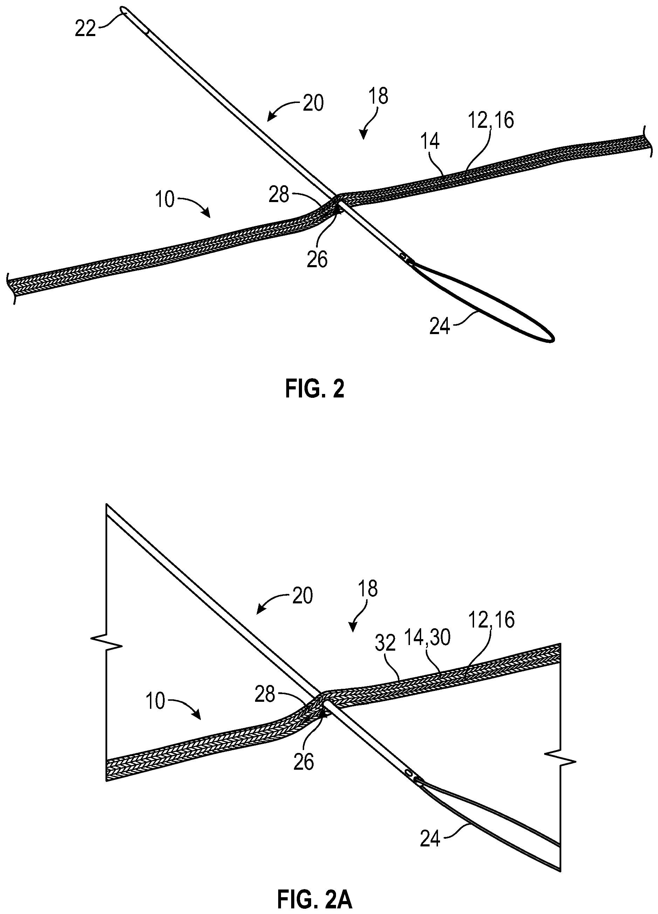

FIGS. 2 and 2A show a system 18 that includes the suture 10 and a needle 20. The needle 20 can include a tip 22 (FIG. 2) and snare loop 24. The suture 10 can include an aperture 26, in addition to the body 12, exterior surface 14 and elongate strand 16.

The needle 20 can have an elongate length from the tip 22 at one end to the snare loop 24 at an opposing end. It is also contemplated that in alternative to a snare loop 24, the needle 20 could simply have an eye or other feature capable of capturing the suture 10 as known in the art. The tip 22 can be configured as a point can be configured to puncture the suture 10 as shown in FIGS. 2 and 2A. This puncture can create the aperture 26 as shown. The aperture 26 can be made at a first portion 28 of the elongate strand 16 of the body 12. The first portion 28 can be a part of the longitudinal length of the suture 10, for example. The aperture 26 can be formed in a manner that does not weaken the suture 10 such as by being positioned between the individual strands that make up the braids of the suture 10. Thus, the aperture 26 can be formed without breaking any of the strands that form the suture 10. The aperture 26 can pass in generally a lateral direction (give or take 60 degrees or so) through the elongate strand 16 relative to the longitudinal extent. The aperture 26 can be enclosed laterally on several sides by the body 12 of the suture 10. Thus, the aperture 26 can have only two openings. One opening of the aperture 26 can be on the first side 30 of the suture and another opening can be on the second opposing side 32 of the suture. As the suture 10 shown in FIGS. 2 and 2A is a ribbon suture, the first side 30 and the second side 32 can be substantially flat and can be arranged generally opposing one another. The aperture 26 can pass through the body 12 from the first opening to the second opening or from the first side 30 to the opposing second side 32.

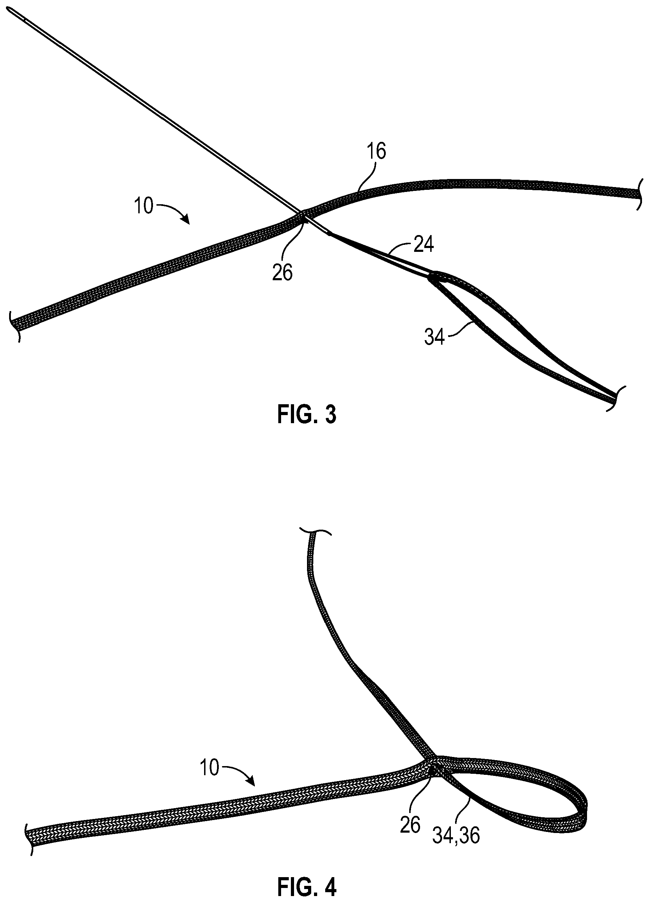

FIG. 3 shows a second part 34 of the suture 10 from where the aperture 26 is located being captured by the snare loop 24. The second part 34 comprises a different portion of the same elongate strand 16 along a different part of the longitudinal length thereof.

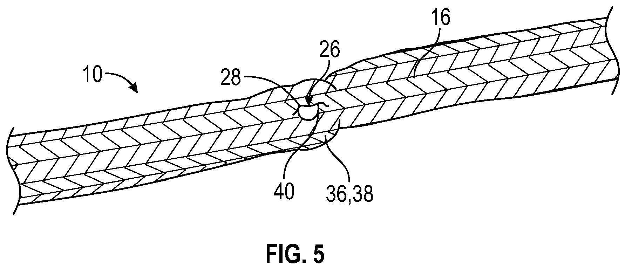

FIG. 4 illustrates the second part 34 of the suture 10 now drawn by the snare loop 24 (FIG. 3) so as to be passed through the aperture 26. Thus, the suture 10 is passed through itself. This forms a loop 36 in the suture 10. Optionally, the loop 36 can be maintained and utilized for connection to another suture or for connection to a surgical device or another element, for example. However, FIG. 5 shows the loop 36 of FIG. 4 has be reduced by continuing to pull the second part 34 through the aperture 26 until loop 36 is closed (i.e., substantially eliminated) leaving a surface feature 38 along the surface 14 of the body 12 of the suture 10. This closed loop configuration passes a second portion 40 of the elongate strand 16 that is adjacent the first portion 28 through the aperture 26, thereby creating the surface feature 38. The surface feature 38 can comprise a bump, a ridge, a fold, a projection or the like. The surface feature 38 can be thought of as a folded portion of the suture 10 where the second portion 40 is folded over and passed through the first portion 28. The surface feature 38 can occur naturally as the loop 36 closes upon itself. The surface feature 38 can be U-shaped, omega shaped or otherwise shaped and can extend around several sides of the aperture 26. The aperture 26 can remain in some instances and shown in FIG. 4. However, in some cases the surface feature 38 can be large enough to obstruct or partially obstruct/cover the aperture 26 at least along the first side 30 of the suture 10. Indeed, as further discussed herein, an element comprising a bead or knot is utilized to enlarge the surface feature 38 to obstruct or substantially obstruct the aperture 26.

As discussed previously, the surface feature 38 can create an area of higher friction along the surface 14 of the suture 10 as compared with the remainder of the surface 14 not having the surface feature 38. This surface feature 38, with a higher degree of friction, can be used to better couple with tissue or better couple with the suture itself or other suture like a ratchet mechanism or zip tie, for example.

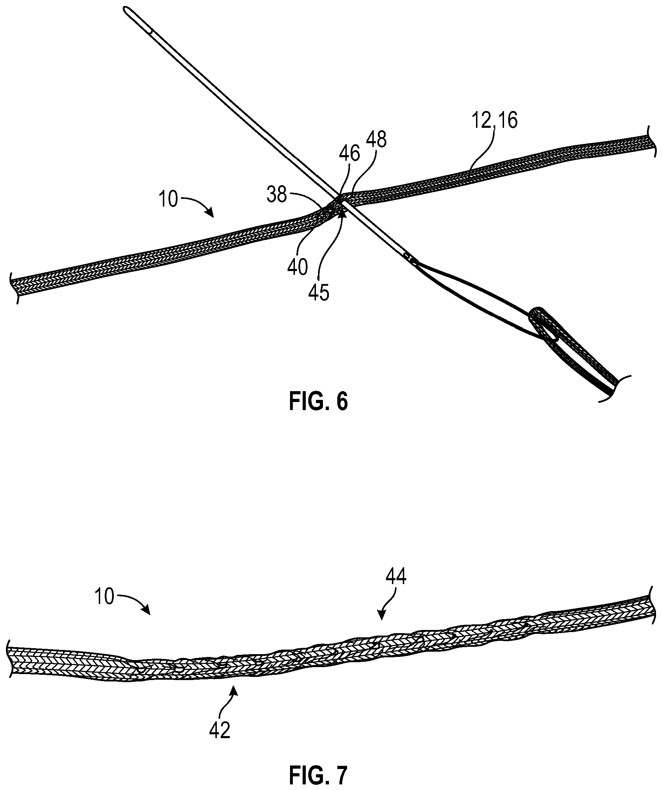

FIGS. 6 and 7 show the methodology described in FIGS. 2-5 repeated on the suture 10 to create a plurality of the surface features 42 (FIG. 7) including the surface feature 38. These plurality of surface features 42 can create a region 44 of relatively higher degree of friction as shown in FIG. 7. This region 44 can have twists or turns of the suture between the plurality of features 42. As shown in FIG. 6, the body 12 can have at least a second aperture 45 therethrough at a third portion 46 of the elongate strand 16. The third portion 46 can be spaced longitudinally along the elongate strand 16 from the second portion 40. A fourth portion 48 of the elongate strand 16 adjacent the third portion 46 can be passed through the second aperture 45 to create at least a second feature along the surface of the body. This second feature is shown as one of the plurality of surface features 42 in FIG. 7.

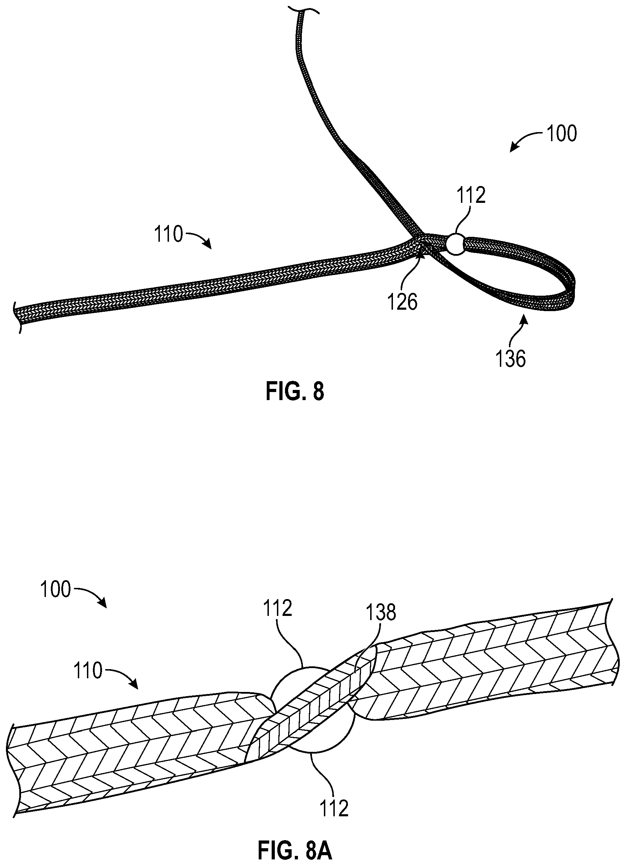

FIGS. 8-11A show examples of various elements that can be captured by the suture using the techniques described above including passing a portion of the suture through an aperture in the suture. The elements contemplated can be anything known in the art for surgical closure that can be passed through or attached to a suture loop. The elements include: a bead(s) and/or a knot(s). The elements may further include: a surgical device(s) (e.g., another suture(s), a ring, a hook, a suture anchor or the like).

FIGS. 8 and 8A show a system 100 according to another example that includes a suture 110 and a bead 112. The suture 110 can be constructed in the manner of the suture 10 previously discussed. FIG. 8 shows the suture 110 punctured in the manner previously described and a portion of the suture 110 passed through an aperture 126 therein. The bead 112 can be placed in a loop 136 formed by passing a portion of the suture 110 through the aperture 126. The bead 112 itself can have a passage or aperture therethrough that allows the suture 110 to pass through the bead 112. The loop 136 can be reduced in size until substantially eliminated in the manner previously described. This results in the suture 110 capturing the bead 112 as shown in FIG. 8A.

The bead 112 can be shaped to enlarge a surface feature 138 that results from reducing and substantially eliminating the loop 136. The surface feature 138 is shown in FIG. 8A. The increased size of the surface feature 138 that results from capturing the bead 112 can result in an area of increased friction along a surface 114 of the suture 110 relative to other area(s) of the surface 114. As discussed previously, the bead 112 can obstruct or partially obstruct the aperture 126 (FIG. 8).

Although the bead 112 of FIGS. 8 and 8A is shown as generally spherical in shape, other shapes are contemplated. For example, the bead 112 can have furrows or channels therein along the surface thereof rather than the passage therethrough as illustrate. These channels or furrows can be designed to receive portions of the suture 110. The bead 112 could be disk, flake, block or otherwise shaped if desired to facilitate different shapes for the surface feature 138.

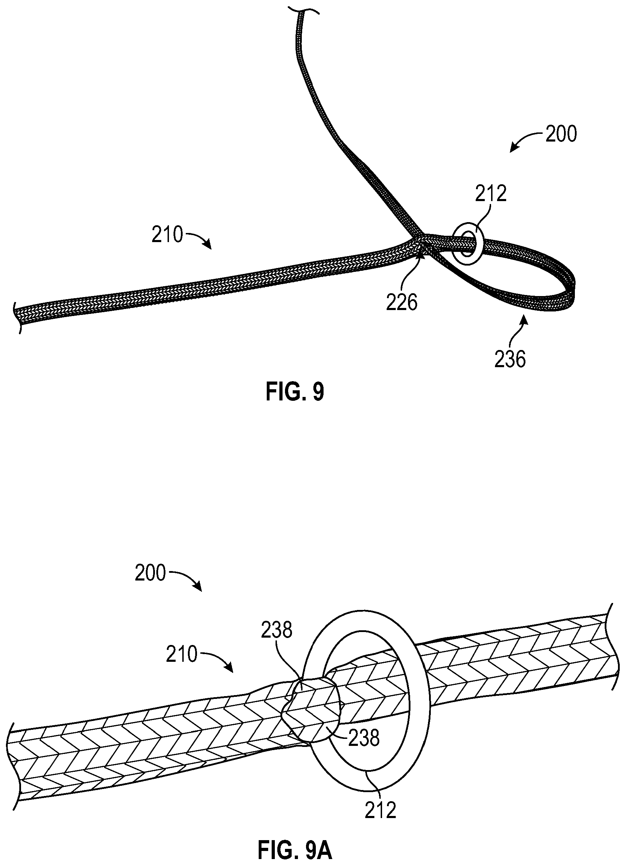

FIGS. 9 and 9A show a system 200 according to another example that includes a suture 210 and a ring 212. The suture 210 can be constructed in the manner of the suture 10 previously discussed. FIG. 9 shows the suture 210 passed through the ring 212 and punctured in the manner previously described. A portion of the suture 210 can be passed through an aperture 226 therein. The ring 212 can be positioned in a loop 236 formed by passing a portion of the suture 210 through the aperture 226. The loop 236 can be reduced in size until substantially eliminated in the manner previously described. This results in the suture 210 capturing the ring 212 as shown in FIG. 9A.

The ring 212 can be shaped to provide a linkage whereby other surgical devices such as additional sutures, etc. can be coupled to the suture 210. The ring 212 can additionally enlarge a surface feature 238 that results from reducing and substantially eliminating the loop 236. The surface feature 238 is shown in FIG. 9A. The ring 212 can obstruct or partially obstruct the aperture 226 (FIG. 9).

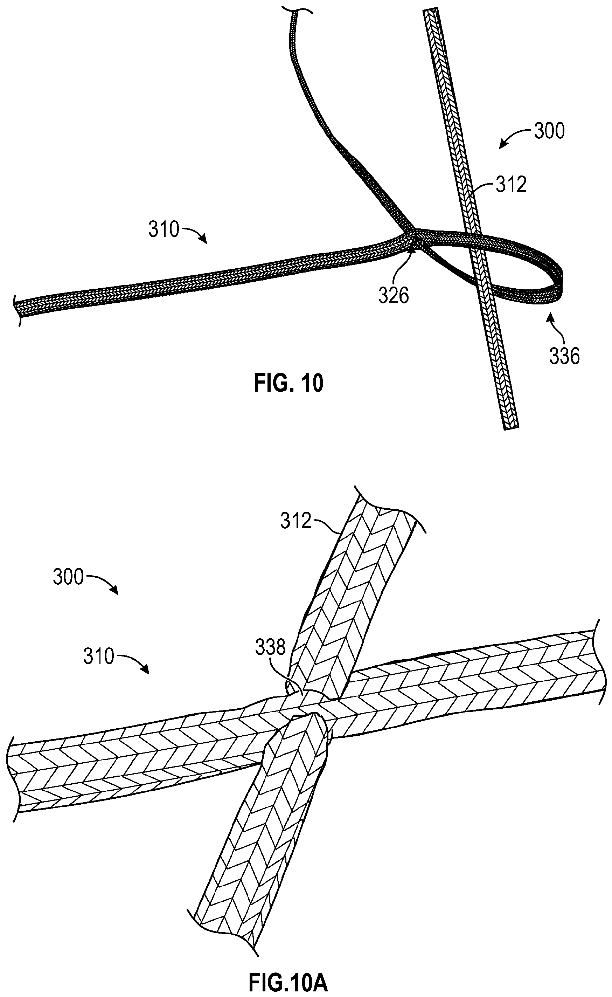

FIGS. 10 and 10A show a system 300 according to another example that includes a first suture 310 and a second suture 312. The first suture 310 and/or the second suture 312 can be constructed in the manner of the suture 10 previously discussed. The first suture 310 can have a same or different construction relative to one another. FIG. 10 shows the first suture 310 punctured in the manner previously described and a portion of the first suture 310 passed through an aperture 326 therein. The second suture 312 can be placed in a loop 336 formed by passing a portion of the first suture 310 through the aperture 326. The loop 336 can be reduced in size until substantially eliminated in the manner previously described. This results in the first suture 310 capturing the second suture 312 as shown in FIG. 10A

The second suture 312 can provide an additional linkage to soft tissue and/or another surgical device such as an anchor. The second suture 312 can be provided with surface features such as those discussed herein (e.g., knots, loops, beads, etc.), which can help prevent the second suture 312 from slipping in the loop 336. The second suture 312 can additionally enlarge a surface feature 338 of the first suture 310 that results from reducing and substantially eliminating the loop 336. The surface feature 338 is shown in FIG. 10A. The second suture 312 can obstruct or partially obstruct the aperture 326 (FIG. 9) of the first suture 310.

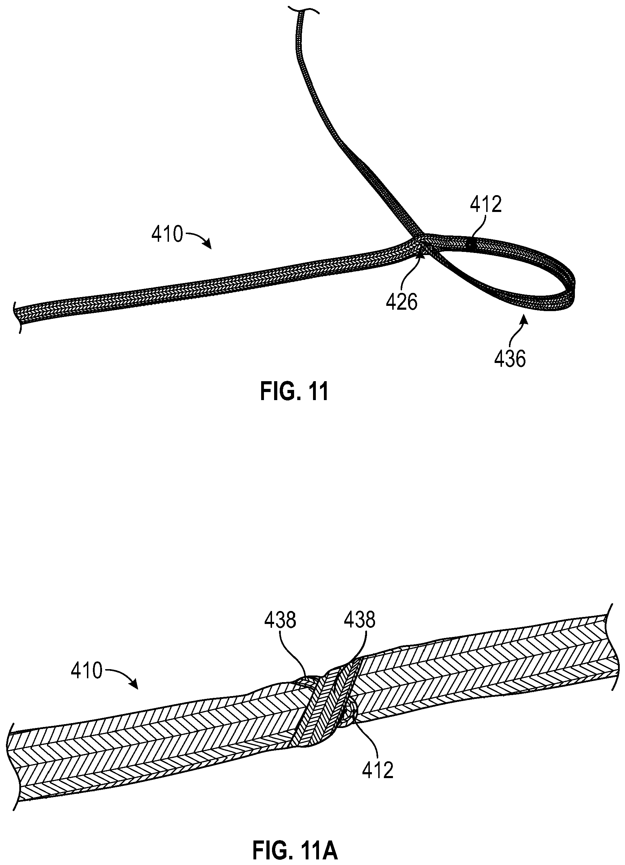

FIGS. 11 and 11A show a suture 410 according to another example. The suture 410 can be constructed in the manner of the suture 10 previously discussed save that the suture 410 can include a knot 412 therein. FIG. 11 shows the suture 410 punctured in the manner previously described and a portion of the suture 410 passed through an aperture 426 therein. The knot 412 can be positioned so as to be placed in and form part of a loop 236 formed by passing a portion of the suture 410 through the aperture 426. The loop 436 can be reduced in size until substantially eliminated in the manner previously described. This results in the suture 410 folded over, capturing or otherwise positioning the knot 412 at or adjacent the aperture 426 as shown in FIG. 11A.

The knot 412 can be shaped in any manner known in the art. Thus, the shape of the knot is purely exemplary. The knot 412 can enlarge a surface feature 438 that results from reducing and substantially eliminating the loop 436. The surface feature 438 is shown in FIG. 11A. The increased size of the surface feature 438 that results from capturing, folding over or otherwise utilizing the knot 412 as part of the closed loop construct can result in an area of increased friction along a surface 414 of the suture 410 relative to other area(s) of the surface 414. As discussed previously, the knot 412 can obstruct or partially obstruct the aperture 426 (FIG. 11).

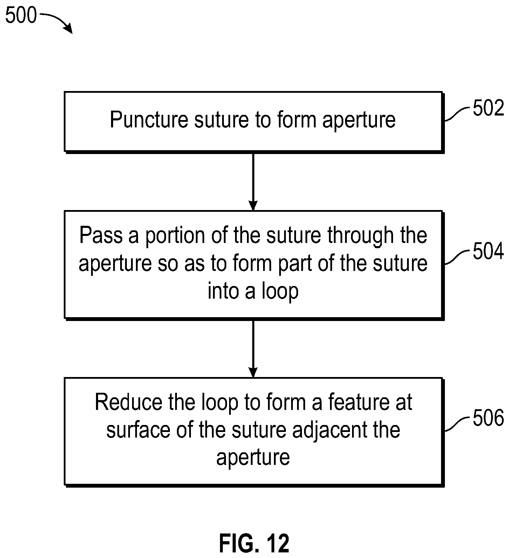

FIG. 12 shows a flowchart for a manufacturing method 500. The method 500 can be used to fabricate OEM suture that can be sold and then utilized in a surgical procedure. The method 500 includes the step of puncturing 502 an elongate body of a suture to form an aperture. The method 500 includes passing 504 a portion of the elongate body through the aperture so as to form part of the elongate body into a loop. The passing the second portion of the elongate body through the aperture so as to form the elongate body into the loop can include capturing the suture in a snare loop that is passed through the aperture. The surgical method 500 includes reducing 506 the loop to form a bump along a surface of the body adjacent the aperture.

The method 500 can optionally include creating a knot in the elongate body and capturing the knot within the loop with the reducing the loop. The method includes capturing an element within the loop with the reducing of the loop as previously described an illustrated. As discussed the element comprises at least one of a knot and a bead, and may further comprise a surgical device or a second suture.

The various stages of method 500 have been described in a particular order for ease of discussion and completeness. However, the various stages of method 500 can be rearranged and/or omitted without departing from the scope of the present disclosure.

### Additional Notes

The above detailed description includes references to the accompanying drawings, which form a part of the detailed description. The drawings show, by way of illustration, specific embodiments in which the invention can be practiced. These embodiments are also referred to herein as "examples." Such examples can include elements in addition to those shown or described. However, the present inventors also contemplate examples in which only those elements shown or described are provided. Moreover, the present inventors also contemplate examples using any combination or permutation of those elements shown or described (or one or more aspects thereof), either with respect to a particular example (or one or more aspects thereof), or with respect to other examples (or one or more aspects thereof) shown or described herein.

In this document, the terms "a" or "an" are used, as is common in patent documents, to include one or more than one, independent of any other instances or usages of "at least one" or "one or more." In this document, the term "or" is used to refer to a nonexclusive or, such that "A or B" includes "A but not B," "B but not A," and "A and B," unless otherwise indicated. In this document, the terms "including" and "in which" are used as the plain-English equivalents of the respective terms "comprising" and "wherein." Also, in the following claims, the terms "including" and "comprising" are open-ended, that is, a system, device, article, composition, formulation, or process that includes elements in addition to those listed after such a term in a claim are still deemed to fall within the scope of that claim. Moreover, in the following claims, the terms "first," "second," and "third," etc. are used merely as labels, and are not intended to impose numerical requirements on their objects.

The scope of the invention should be determined with reference to the appended claims.

## Claims

1. A suture (10, 110, 210, 310, 410) for a surgical procedure comprising;
a body (12) comprising an elongate strand (16), the body (12) having an aperture (26, 126, 226, 326, 426) therethrough at a first portion (28) of the elongate strand (16), wherein a second portion (40) of the elongate strand (16) adjacent the first portion (28) is passed through the aperture (26, 126, 226, 326, 426) to create a feature (38) comprising a bump along an exterior surface (14) of the body (12); and
an element captured by the feature, wherein the element comprises at least one of a knot and a bead.

2. The suture (10, 110, 210, 310, 410) of claim 1, wherein the body (12) has at least a second aperture (45) therethrough at a third portion (46) of the elongate strand (16), wherein the third portion (46) is spaced longitudinally along the elongate strand (16) from the second portion (40), and wherein a fourth portion (48) of the elongate strand (16) adjacent the third portion (46) is passed through the at least the second aperture (45) to create at least a second feature along the exterior surface (14) of the body.

3. The suture (10, 110, 210, 310, 410) of any one of claims 1-2, wherein the second portion (40) is folded over and passed through at least a part of the first portion (28).

4. The suture (10, 110, 210, 310, 410) of any one of claims 1-3, wherein the body (12) is formed of a plurality of braided strands that together form the elongate strand (16), and wherein the exterior surface (14) of the body (12) is substantially flat.

5. The suture (10, 110, 210, 310, 410) of any of claims 1-4, wherein the element further comprises at least one of a surgical device or a second suture (312).

6. A system (18) comprising:
the suture (10, 110, 210, 310, 410) of any of claims 1-5; and
a needle (20) having a first end configured to puncture the suture (10, 110, 210, 310, 410) to create the aperture (26, 126, 226, 326, 426) and a second part having a snare loop (24), eye or other feature configured to capture the suture (10, 110, 210, 310, 410), wherein the needle (20) is configured to pass through the aperture (26, 126, 226, 326, 426) to pass the suture (10, 110, 210, 310, 410) through the aperture (26, 126, 226, 326, 426).

7. A method of manufacturing a suture (10, 110, 210, 310, 410) comprising:
providing a suture (10, 110, 210, 310, 410) comprising an elongate body (12);
puncturing (502) the elongate body (12) of the suture (10, 110, 210, 310, 410) to form an aperture (26, 126, 226, 326, 426) therein;
passing (504) a portion of the elongate body (12) through the aperture (26, 126, 226, 326, 426) so as to form part of the elongate body (12) into a loop; and
reducing (506) the loop to form a feature (38) comprising a bump along an exterior surface (14) of the body (12) adjacent the aperture (26, 126, 226, 326, 426),
wherein the method further comprises capturing an element within the loop with the reducing of the loop, such that the element is captured by the feature when the loop is reduced, wherein the element comprises at least one of a knot and a bead .

8. The method of claim 7, wherein the element further comprises at least one of a surgical device or a second suture (312).

9. The method of any one of claims 7-8, wherein passing the portion of the elongate body (12) through the aperture (26, 126, 226, 326, 426) so as to form the elongate body (12) into the loop includes capturing the suture (10, 110, 210, 310, 410) in a snare loop (24), eye or other feature that is passed through the aperture (26, 126, 226, 326, 426).

## Patentansprüche

1. Naht (10, 110, 210, 310, 410) für einen chirurgischen Eingriff, umfassend;
einen Körper (12), der einen länglichen Strang (16) umfasst, wobei der Körper (12) eine Öffnung (26, 126, 226, 326, 426) dort hindurch an einem ersten Abschnitt (28) des länglichen Strangs (16) aufweist, wobei ein zweiter Abschnitt (40) des länglichen Strangs (16) benachbart zu dem ersten Abschnitt (28) durch die Öffnung (26, 126, 226, 326, 426) geführt ist, um ein Merkmal (38) zu erschaffen, das eine Erhebung entlang einer Außenfläche (14) des Körpers (12) umfasst; und
ein Element, das durch das Merkmal erfasst wird, wobei das Element zumindest eines von einem Knoten und einem Wulst umfasst.

2. Naht (10, 110, 210, 310, 410) nach Anspruch 1, wobei der Körper (12) zumindest eine zweite Öffnung (45) dort hindurch an einem dritten Abschnitt (46) des länglichen Strangs (16) aufweist, wobei der dritte Abschnitt (46) längs entlang des länglichen Strangs (16) von dem zweiten Abschnitt (40) beabstandet ist, und wobei ein vierter Abschnitt (48) des länglichen Strangs (16) benachbart zu dem dritten Abschnitt (46) durch die zumindest zweite Öffnung (45) geführt ist, um zumindest ein zweites Merkmal entlang der Außenfläche (14) des Körpers zu erschaffen.

3. Naht (10, 110, 210, 310, 410) nach einem der Ansprüche 1-2, wobei der zweite Abschnitt (40) umgefaltet und durch zumindest einen Teil des ersten Abschnittes (28) geführt ist.

4. Naht (10, 110, 210, 310, 410) nach einem der Ansprüche 1-3, wobei der Körper (12) aus einer Vielzahl von geflochtenen Strängen gebildet ist, die zusammen den länglichen Strang (16) bilden, und wobei die Außenfläche (14) des Körpers (12) im Wesentlichen flach ist.

5. Naht (10, 110, 210, 310, 410) nach einem der Ansprüche 1-4, wobei das Element ferner zumindest eines von einer chirurgischen Vorrichtung oder einer zweiten Naht (312) umfasst.

6. System (18), umfassend:
die Naht (10, 110, 210, 310, 410) nach einem der Ansprüche 1-5; und
eine Nadel (20) mit einem ersten Ende, das dazu konfiguriert ist, die Naht (10, 110, 210, 310, 410) zu punktieren, um die Öffnung (26, 126, 226, 326, 426) zu erschaffen, und einem zweiten Teil mit einer Schlingenschlaufe (24), einer Öse oder einem anderen Merkmal, die/das dazu konfiguriert ist, die Naht (10, 110, 210, 310, 410) zu erfassen, wobei die Nadel (20) dazu konfiguriert ist, durch die Öffnung (26, 126, 226, 326, 426) geführt zu werden, um die Naht (10, 110, 210, 310, 410) durch die Öffnung (26, 126, 226, 326, 426) zu führen.

7. Verfahren zum Herstellen einer Naht (10, 110, 210, 310, 410), umfassend:
Bereitstellen einer Naht (10, 110, 210, 310, 410), umfassend einen länglichen Körper (12);
Punktieren (502) des länglichen Körpers (12) der Naht (10, 110, 210, 310, 410), um eine Öffnung (26, 126, 226, 326, 426) darin zu bilden;
Führen (504) eines Abschnittes des länglichen Körpers (12) durch die Öffnung (26, 126, 226, 326, 426), um einen Teil des länglichen Körpers (12) zu einer Schlaufe zu bilden; und
Reduzieren (506) der Schlaufe, um ein Merkmal (38) zu bilden, das eine Erhebung entlang einer Außenfläche (14) des Körpers (12) benachbart zu der Öffnung (26, 126, 226, 326, 426) umfasst,
wobei das Verfahren ferner Erfassen eines Elements innerhalb der Schlaufe mit dem Reduzieren der Schlaufe umfasst, sodass das Element durch das Merkmal erfasst wird, wenn die Schlaufe reduziert wird, wobei das Element zumindest eines von einem Knoten und einem Wulst umfasst.

8. Verfahren nach Anspruch 7, wobei das Element ferner zumindest eines von einer chirurgischen Vorrichtung oder einer zweiten Naht (312) umfasst.

9. Verfahren nach einem der Ansprüche 7-8, wobei das Führen des Abschnittes des länglichen Körpers (12) durch die Öffnung (26, 126, 226, 326, 426), um den länglichen Körper (12) zu der Schlaufe zu bilden, Erfassen der Naht (10, 110, 210, 310, 410) in einer Schlingenschlaufe (24), einer Öse oder einem anderen Merkmal, die/das durch die Öffnung (26, 126, 226, 326, 426) geführt ist, beinhaltet.

## Revendications

1. Suture (10, 110, 210, 310, 410) pour une intervention chirurgicale, comprenant ;
un corps (12) comprenant un brin allongé (16), le corps (12) présentant une ouverture (26, 126, 226, 326, 426) en son travers au niveau d'une première partie (28) du brin allongé (16), une deuxième partie (40) du brin allongé (16) adjacente à la première partie (28) étant passée à travers l'ouverture (26, 126, 226, 326, 426) de manière à créer une caractéristique (38) comprenant une bosse le long d'une surface extérieure (14) du corps (12) ; et
un élément capturé par la caractéristique, ledit élément comprenant au moins l'un d'un nœud et d'une bille.

2. Suture (10, 110, 210, 310, 410) selon la revendication 1, ledit corps (12) comportant au moins une seconde ouverture (45) en son travers au niveau d'une troisième partie (46) du brin allongé (16), ladite troisième partie (46) étant espacée longitudinalement le long du brin allongé (16) de la deuxième partie (40), et une quatrième partie (48) du brin allongé (16) adjacente à la troisième partie (46) étant passée à travers ladite au moins seconde ouverture (45) de manière à créer au moins une seconde caractéristique le long de la surface extérieure (14) du corps.

3. Suture (10, 110, 210, 310, 410) selon l'une quelconque des revendications 1 à 2, ladite deuxième partie (40) étant repliée et passée à travers au moins une partie de la première partie (28).

4. Suture (10, 110, 210, 310, 410) selon l'une quelconque des revendications 1 à 3, ledit corps (12) étant formé d'une pluralité de brins tressés qui forment ensemble le brin allongé (16), et ladite surface extérieure (14) du corps (12) étant sensiblement plate.

5. Suture (10, 110, 210, 310, 410) selon l'une quelconque des revendications 1 à 4, ledit élément comprenant en outre au moins un dispositif chirurgical ou une seconde suture (312).

6. Système (18) comprenant :
la suture (10, 110, 210, 310, 410) selon l'une quelconque des revendications 1 à 5 ; et
une aiguille (20) possédant une première extrémité conçue pour perforer la suture (10, 110, 210, 310, 410) de manière à créer l'ouverture (26, 126, 226, 326, 426) et une seconde partie présentant une boucle en collet (24), un œil ou une autre caractéristique conçue pour capturer la suture (10, 110, 210, 310, 410), ladite aiguille (20) étant conçue pour passer à travers l'ouverture (26, 126, 226, 326, 426) de manière à faire passer la suture (10, 110, 210, 310, 410) à travers l'ouverture (26, 126, 226, 326, 426).

7. Procédé de fabrication d'une suture (10, 110, 210, 310, 410), comprenant :
la fourniture d'une suture (10, 110, 210, 310, 410) comprenant un corps allongé (12) ;
la perforation (502) du corps allongé (12) de la suture (10, 110, 210, 310, 410) de manière à former une ouverture (26, 126, 226, 326, 426) en son sein ;
le passage (504) d'une partie du corps allongé (12) à travers l'ouverture (26, 126, 226, 326, 426) de manière à former une partie du corps allongé (12) en une boucle ; et
la réduction (506) de la boucle de manière à former une caractéristique (38) comprenant une bosse le long d'une surface extérieure (14) du corps (12) adjacente à l'ouverture (26, 126, 226, 326, 426),
ledit procédé comprenant en outre la capture d'un élément à l'intérieur de la boucle avec la réduction de la boucle, de sorte que l'élément soit capturé par la caractéristique lorsque la boucle est réduite, ledit élément comprenant au moins l'un d'un nœud et d'une bille .

8. Procédé selon la revendication 7, ledit élément comprenant en outre au moins un dispositif chirurgical ou une seconde suture (312).

9. Procédé selon l'une quelconque des revendications 7 à 8, ledit passage de la partie du corps allongé (12) à travers l'ouverture (26, 126, 226, 326, 426) de manière à former le corps allongé (12) dans la boucle comprenant la capture de la suture (10, 110, 210, 310, 410) dans une boucle en collet (24), un œil ou une autre caractéristique qui est passée à travers l'ouverture (26, 126, 226, 326, 426).
